# EUROPEAN PATENT APPLICATION

(11) **EP 3 492 090 A1**
(43) Date of publication of application: **05.06.2019**
(21) Application number: 17836703.3
(22) Date of filing: 11.07.2017
(51) Int. Cl.: A61K 35/12, A61K 35/28, A61P 1/16, A61P 9/10, A61P 13/12, C12N 5/077

(54) **METHOD FOR PREPARING THERAPEUTIC AGENT**

(30) Priority: 01.08.2016 JP 2016151555
(71) Applicant: Yamaguchi University, Yamaguchi 753-8511 (JP); Shibuya Corporation, Kanazawa-shi, Ishikawa 920-8681 (JP)
(72) Inventor: SAKAIDA Isao, Ube-shi Yamaguchi 755-8505 (JP); TAKAMI Taro, Ube-shi Yamaguchi 755-8505 (JP); YONEDA Kenji, Ishikawa 920-8681 (JP)
(74) Representative: Mewburn Ellis LLP
(86) International application number: PCT/JP2017/025279
(87) International publication number: WO 2018/025596

(57) **Abstract**

In an operating room 8, approximately 40 mL of a bone marrow fluid 10 of a patient is sampled and housed in a collection tube 11. Then, the collection tube 11 is conveyed into an isolator 2 in a sterile state. In the isolator 2, the bone marrow fluid 10 in the collection tube 11 is pipetted into test tubes 13 and culture tube 14, etc.

To the bone marrow fluid 10 in the tubes 14, an erythrocyte sedimentation agent is added. After thus precipitating erythrocytes, the supernatant is collected. From the collected supernatant, a bone marrow cell-containing fraction for liver regeneration is concentrated. The obtained concentrate is pipetted into flasks 5 (culture containers). Then, the flasks 5 are conveyed into an incubator 4 to start the cell-culture.

From the isolator 1, test tubes 13 and 16 are taken out. The bone marrow fluid in the test tubes 13 and 16 and the concentrate are subjected to a safety test, etc.

The safety of the bone marrow fluid 10 sampled from the patient can be tested immediately after starting the cell-culture.

## Description

### [Technical Field]

The present invention relates to a method for preparing a therapeutic agent and more particularly, it relates to a method for preparing a therapeutic agent used for treatments for diseases such as hepatic failures, kidney failures, cerebral infarction, etc.

### [Background Art]

Conventionally, treatments for organs and tissues by sampling a bone marrow fluid of a patient and by returning Mesenchymal stem cells in the bone marrow fluid into the body of the patient have been tried. For example, a treatment method in which the bone marrow fluid of a patient is sampled, washed/concentrated and then, it is returned to a vein of the patient in order to treat a patient having a hepatic failure has been proposed, and moreover, a method for preparing the therapeutic agent used for the treatment method has been also proposed (Patent Document 1, for example).

In the method for preparing in Patent Document 1, a transfusion fluid (therapeutic agent) is prepared by sampling 400 mL of the bone marrow fluid from a patient and by washing/concentrating the bone marrow fluid, and the treatment effect is obtained by returning the transfusion fluid (therapeutic agent) into the vein of the patient.

### [Prior Art Documents]

### [Patent Document]

[Patent Document 1] Japanese Patent No. 4752058

### [Non-Patent Document]

[Non-Patent Document 1] Biochemistry, Vol. 84, pp. 707 - 711, 2012

### [Summary of Invention]

### [Problems to be Solved]

In the method for preparing a therapeutic agent in Patent Document 1, general anesthesia needs to be performed on a patient in order to sample a required amount (400 mL) of a bone marrow fluid, and since sampling time is long, there is a problem that a physical burden on the patient is large.

Thus, a treatment method of obtaining a treatment effect equivalent to that of Patent Document 1 by culturing a small amount (approximately 30 mL) of the bone marrow fluid of the patient is sampled and by returning the incubated cell to the patient has been conventionally proposed (Non-Patent Document 1). In this treatment method, the bone marrow fluid sampled from the patient is assumed not to be contaminated by viruses, but safety of the bone marrow fluid sampled from the patient has not been particularly considered. Thus, if the bone marrow fluid sampled from the patient is contaminated by the viruses, the subsequent culture work itself of the bone marrow fluid becomes useless, which is a problem.

### [Means for Solving the Problems]

In view of the aforementioned circumstances, the present invention is a method for preparing a therapeutic agent in which cells are incubated after required processing is performed on the cells sampled from a human body in an isolator maintained in a sterile state, characterized by including:
a process of conveying a collection container housing the cells sampled from the human body into the isolator, a process of pipetting the cells in the collection container into test containers and culture containers in the isolator in the sterile state, a process of conveying the culture containers housing the cells into an incubator and of starting the cell-culture, and a process of conducting required tests on the cells in the test containers.

### [Advantageous Effect of the Invention]

According to such constitution, safety of the cells sampled from the human body can be tested, and contamination of the cells incubated in the incubator can be reliably prevented.

### [Brief Description of the Drawings]

Figure 1 is a schematic configuration diagram illustrating an embodiment of the present invention.
Figure 2 is a view illustrating a process using an isolator system illustrated in Figure 1.

### [Mode for Carrying out the Invention]

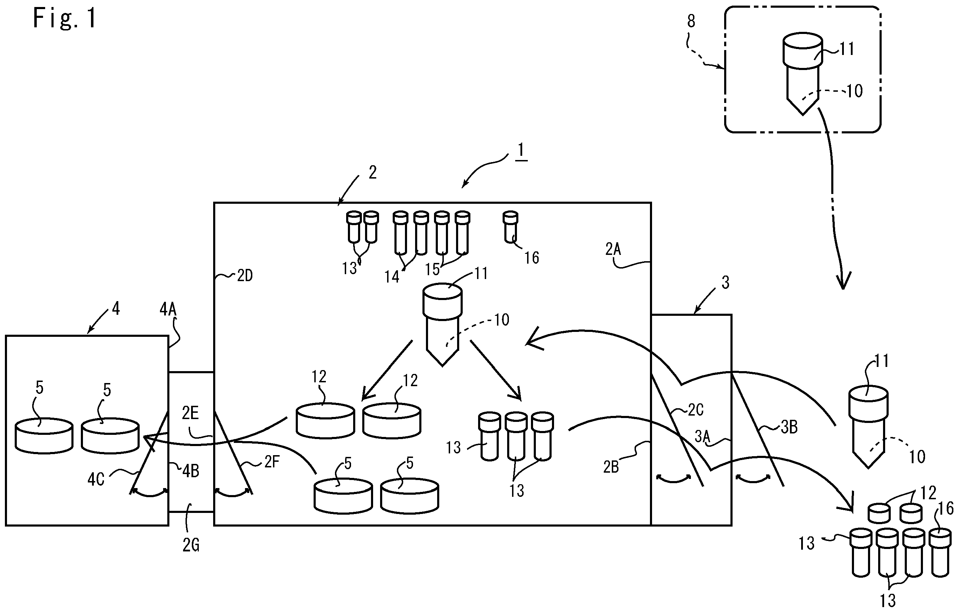

Hereinafter, the present invention will be described on the basis of an illustrated embodiment, and in Figure 1, reference character 1 denotes an isolator system, and cells are incubated by using this isolator system 1, and a therapeutic agent is prepared for a patient having a hepatic failure, and safety of a bone marrow fluid (cells) sampled from the patent is tested.

The isolator system 1 includes an isolator 2 in which an inside thereof is maintained in a sterile state and required works are performed, a pass box 3 connected to a side wall 2A of this isolator 2, and an incubator 4 detachably provided on the isolator 2 and in which the cell-culture is performed in the sterile state.

In one side wall 2A of the isolator 2, an opening portion 2B is formed, and an opening/closing door 2C for opening/closing the opening portion 2B is provided. And the pass box 3 is connected to the side wall 2A by covering the opening portion 2B and the opening/closing door 2C from outside. An opening portion 3A is formed in a side wall of the pass box 3 facing the opening portion 2B, and an opening/closing door 3B for opening/closing the opening portion 3A is provided.

By opening the opening/closing door 3B of the pass box 3, a container such as a tube with lid and any other instruments can be entered into/taken out of the pass box 3 through the opening portion 3A, the opening portion 3A is closed by the opening/closing door 3B after the container or the like is conveyed into and outer-surface decontamination of the container or the like is performed in the pass box 3 and then, by opening the opening/closing door 2C of the isolator 2, the container or the like can be entered into/taken out between the isolator 2 and the pass box 3 through the opening portion 2B.

An opening portion 2E is also formed in a side wall 2D of the isolator 2 on a side opposite to the pass box 3, and an opening/closing door 2F for opening/closing this opening portion 2E is provided. Moreover, a connection port 2G for connection by holding airtightness of an incubator 4 is provided by covering the opening portion 2E on an outer side of the side wall 2D of the isolator 2. In a state where the incubator 4 is connected at a position of the connection port 2G, the container or the like can be conveyed from inside the isolator 2 into the incubator 4 or from inside the incubator 4 into the isolator 2 through the opening portion 2E.

An opening portion 4B is formed in a wall portion 4A on a front surface of the incubator 4, and an opening/closing door 4C for opening/closing the opening portion 4B from an outer side is provided. When necessary, the entire incubator 4 is moved to a position adjacent to the isolator 2 and is coupled with the connection port 2G of the isolator 2 so that connection can be made while airtightness is held.

Then, by opening the opening/closing doors 2F and 4C in a state where the incubator 4 is connected to a position of the connection port 2G of the isolator 2 as illustrated in Figure 1, the inside of the isolator 2 and the inside of the incubator 4 communicate with each other through the connection port 2G, and in that state, a flask 5 as the culture container is conveyed from inside the isolator 2 into the incubator 4.

On the side wall of the front surface of the isolator 2, a plurality of gloves, not shown, is provided, and a transparent window through which the inside can be checked is provided. And a worker inserts both hands into the gloves and performs required works in the isolator 2 and performs an opening/closing work of the opening/closing doors 2C and 2F, conveying-in/out works of the container, instruments and the like from the pass box 3 into the isolator 2, a conveying-in work of the container from inside the isolator 2 into the incubator 4 and the like.

Into the isolator 2 as a sterile work chamber and the inside of the pass box 3 connected thereto, a decontamination gas is supplied by a decontaminating device, not shown, at required time in the state where the opening/closing doors 2C, 2F, and 3B are closed or in a state where the opening/closing doors 2F and 3B are closed and the opening/closing door 2C is opened. As a result, the insides of the isolator 2 and the pass box 3 are decontaminated and maintained in the sterile state.

Moreover, the decontamination gas is supplied by the decontaminating device, not shown, at required time also into the incubator 4, and the inside of the incubator 4 is decontaminated and maintained in the sterile state by supplying the decontamination gas in the state where the opening/closing door 4C is closed. Moreover, when the isolator 2 and the incubator 4 are coupled with each other, the incubator is coupled with the connection port 2G of the isolator 2, and by introducing the decontamination gas into the connection port 2G for sterilization in the state where the opening/closing doors 2F and 4C are closed, the isolator 2 and the incubator 4 can be connected in a sterile manner.

Thus, this embodiment is characterized in that a bone marrow fluid 10 is sampled from a patient as cells to be incubated, it is incubated by the isolator system 1, and a therapeutic agent is prepared, and safety of the bone marrow fluid is tested immediately after start of the cell-culture.

That is, the cell-culture (preparation of the therapeutic agent) and the test are performed by the following work processes.

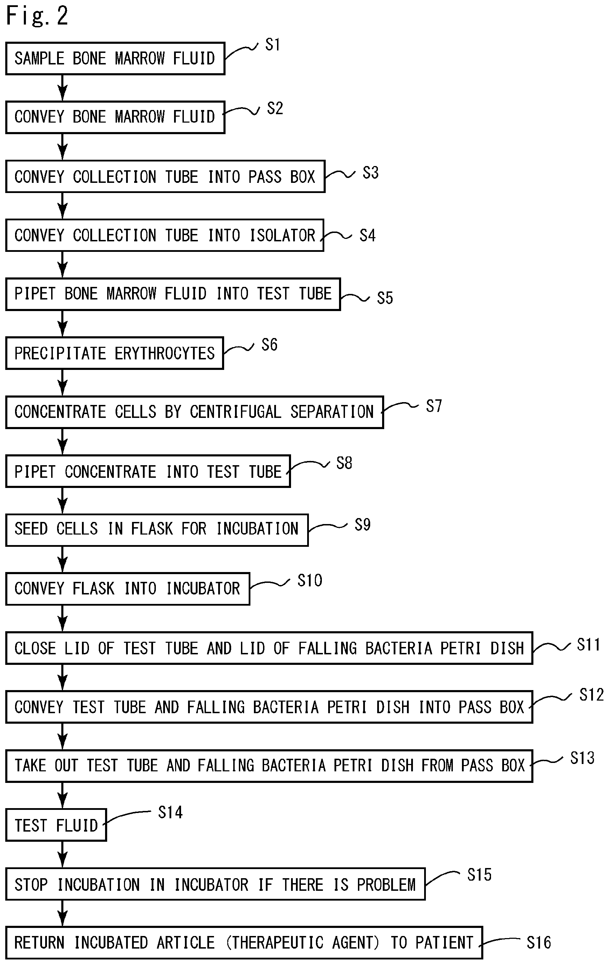

First, in an operating room 8 in a hospital, 30 to 50 mL of the bone marrow fluid 10 is sampled from an iliac bone of a patient with a hepatic failure under local anesthesia, it is placed in a collection tube 11 (collection container) with lid and conveyed to an installation place of the isolator system 1 (see Figure 1 and S1 and S2 in Figure 2). In the aforementioned Patent Document 1, 400 mL of the bone marrow fluid 10 was sampled from a patient under general anesthesia, while in this embodiment, since a sampled amount is small (approximately 30 to 50 mL), the bone marrow fluid 10 is sampled in a state where the patient is under local anesthesia.

After that, in the isolator system 1, the worker performs the work in accordance with the following procedure. At a stage before the collection tube 11 housing the bone marrow fluid 10 is conveyed to the isolator system 1, the incubator 4 is connected at the position of the connection port 2G of the isolator 2 in a sterile manner as illustrated in Figure 1. Moreover, each of the opening/closing doors 2C, 2F, 4B, and 3B is closed, and insides of the isolator 2, the incubator 4, and the pass box 3 are decontaminated in advance by the decontamination gas and in the sterile state. Moreover, inside the isolator 2, instruments required for the preparation work such as a falling bacteria Petri dish 12 having a required size, the tubes 13 to 15 with lid and the like have been conveyed in advance, and those instruments are also decontaminated by the decontamination gas or the like in advance.

In this state, the worker first opens the opening/closing door 3B of the pass box 3, conveys the collection tube 11 housing the bone marrow fluid 10 into the pass box 3 through the opening portion 3A and then, closes the opening/closing door 3B. After this, since the decontamination gas is supplied into the pass box 3 from the decontaminating device, an outer surface of the collection tube 11 is decontaminated, and aeration of the decontamination gas is performed (Figure 1, S3 in Figure 2). Decontamination is not limited to the decontamination by the decontamination gas, but the outer surface of the collection tube 11 may be wiped with alcohol.

After this, the worker inserts both hands into the gloves of the isolator 2 and performs the following works in the isolator 2.

First, the opening/closing door 2C is opened, the collection tube 11 housing the bone marrow fluid 10 is conveyed from the pass box 3 into the isolator 2, and the opening/closing door 2C is closed (Figure 1, S4 in Figure 2) .

After that, the one falling bacteria Petri dish 12 in the isolator 2 is placed on the depth on the right, the second falling bacteria Petri dish 12 is placed on the depth on the left and their lids are opened (Figure 1) .

Subsequently, the bone marrow fluid 10 in the collection tube 11 is pipetted into a plurality of test tubes 13 with lid having a capacity of 10 mL by 1 mL each (Figure 1, S5 in Figure 2). These tubes 13 housing the bone marrow fluid 10 is used at the test of safety or the like of the bone marrow fluid 10 which will be described later.

Subsequently, the bone marrow fluid 10 in the collection tube 11 is pipetted into a tube 14 having a capacity of 75 mL, and erythrocytes are precipitated by using PBS (phosphate-buffered saline), HES (erythrocyte sedimentation agent) and the like (S6 in Figure 2).

Subsequently, a cell-containing fraction is taken out by centrifuging by a centrifugal separator, not shown, installed in the isolator 2 and is put into a 50-mL tube 15 so as to separate Mesenchymal stem cells (S7 in Figure 2).

As a result, a concentrate in which the Mesenchymal stem cells (bone marrow cell-containing fraction for liver regeneration) are concentrated is created in the tube 15.

After that, 1 mL of the concentrate is put into a tube 16 having a capacity of 10 mL. This is for cell-count at the test which will be described later (S8 in Figure 2).

Furthermore, the remaining concentrate is pipetted into a plurality of the flasks 5 having culture mediums therein (S9 in Figure 2). As a result, the cells are seeded in the culture medium in the plurality of flasks 5.

After that, after the opening/closing doors 2F and 4C are opened, the flasks 5 are conveyed from the isolator 2 into the incubator 4 and then, the opening/closing doors 2F and 4C are closed. As a result, the cell-culture of the bone marrow fluid 10 is started by the flasks 5 in the incubator 4 (Figure 1, S10 in Figure 2).

On the other hand, immediately after this, in the isolator 2, the lids of the plurality of tubes 13 into which the bone marrow fluid 10 was pipetted at first by 1 mL each and lids of the two falling bacteria Petri dishes 12 and 12 are closed (S11 in Figure 2).

After that, the opening/closing door 2C of the isolator 2 is opened, and the two falling bacteria Petri dishes 12 and 12, the tubes 13, and the tube 16 for cell count are conveyed into the pass box 3 (S12 in Figure 2). The bone marrow fluid 10 in these tubes 13 and the concentrate in the tube 16 are used for virus check in the test which will be described later and for cell count.

Subsequently, after the opening/closing door 2C is closed, the opening/closing door 3B of the pass box 3 is opened, and the two falling bacteria Petri dishes 12 and 12, the tubes 13 and the tube 16 for virus check and for cell count in the pass box 3 are taken out of the pass box 3 (Figure 1, S13 in Figure 2).

And after that, the bone marrow fluid 10 in the tubes 13 and the concentrate in the tube 16 taken out of the pass box 3 are subjected to various tests as below (S14 in Figure 2).

First, an appearance test of the bone marrow fluid 10 in the tubes 13 is conducted. This appearance test is conducted through visual check by the worker that there is no abnormality with the appearance.

Subsequently, the number of cells in the bone marrow fluid 10 is tested. This test is to make measurement by an automatic blood-cell counting device, and the bone marrow fluid 10 in the tubes 13 and the concentrate in the tube 16 are test targets. The number of cells is calculated by an improved Neubauer calculation board and a microscope as necessary.

Moreover, a test is conducted for Viability. In this test, calculation is made by a trypan blue dying method by using the improved Neubauer calculation board and the microscope.

The test targets here are the bone marrow fluid 10 in the tubes 13. The aforementioned tests are conducted immediately after the flasks 5 are conveyed into the incubator 4, and the cell-culture is started.

Moreover, a sterility test is conducted for the bone marrow fluid 10 in the tubes 13. This test uses an enrichment culture method, and a bacteria test is conducted after the cell-culture by using an exclusive culture device (BACTEC).

Subsequently, an endotoxin test is conducted by a colorimetry method on the bone marrow fluid 10 in the tubes 13.

In this embodiment, the aforementioned tests are conducted. As the result of the tests, if it is determined that there is a problem with safety of the bone marrow fluid 10 sampled from the patient and the concentrate, the cell-culture in the flask 5 in the incubator 4 is stopped, and the flask 5 is taken out of the incubator 4 and discarded (S15 in Figure 2).

On the other hand, as the result of the aforementioned tests, if it is determined that there is no problem with the safety of the bone marrow fluid 10 sampled from the patient and the concentrate, the cell-culture by the flask 5 in the incubator 4 is continued.

Then, after that, by completing subculture of the cells in the incubator 4, the therapeutic agent (incubated article) as a completed product is prepared. Then, the container housing the therapeutic agent is taken out of the incubator 4 and then, carried into the operating room in the hospital and the therapeutic agent is returned into the body through the vein of the patient. As a result, the treatment effect of the patient with a hepatic failure is obtained (S16 in Figure 2) .

According to the embodiment as above, a sample for tests equivalent to the cells incubated in the incubator 4 can be created in the isolator 2 in the sterile state. Thus, occurrence of contamination in the cells in the flask 5 incubated in the incubator 4 can be reliability prevented. Moreover, immediately after the start of the cell-culture in the incubator 4, safety of the bone marrow fluid 10 sampled from the patient can be reliably tested by using the aforementioned test samples.

Moreover, in the method for preparing in the aforementioned Patent Document 1, since 400 mL of the bone marrow fluid is sampled in the state where the patient is under general anesthesia, there was a problem that a physical burden on the patient was large. On the other hand, in this embodiment, it is only necessary that a small amount (approximately 30 to 50 mL) of the bone marrow fluid 10 is sampled in the state where the patient is under local anesthesia. Thus, in this embodiment, the physical burden on the patient when the bone marrow fluid is sampled can be reduced as compared with the conventional cases, and time required for sampling of the bone marrow fluid 10 can be also drastically shortened.

In the aforementioned embodiment, after the tubes 13 and 16 as the test containers are taken out to the outside through the pass box 3, the required tests are conducted on the bone marrow fluid 10 and the concentrate in the tubes 13 and 16, but the safety or the like of the bone marrow fluid 10 and the like in the tubes 13 and 16 can be also tested in the isolator 2.

Moreover, in the aforementioned embodiment, the case where the cells of the bone marrow fluid 10 sampled from the patient are incubated is described, but the present invention can be also applied to a case where cells sampled from a human body other than the bone marrow fluid are incubated.

Moreover, in the aforementioned embodiment, a cell count, viability, sterility and the like are exemplified as test items, but other test items for checking safety of the cells can be also set.

### [Reference Signs List]

- 1: isolator system
- 2: isolator
- 4: incubator
- 5: flask (culture container)
- 10: bone marrow fluid (cells sampled from human body)
- 11: collection tube (collection container)
- 13, 16: tube (test container)
- 14, 15: tube (culture container)

## Claims

1. A method for preparing a therapeutic agent in which cells are incubated after required processing is performed on the cells sampled from a human body in an isolator maintained in a sterile state, **characterized by** comprising:
a step of conveying a collection container housing the cells sampled from the human body into the isolator;
a step of pipetting the cells in the collection container into test containers and culture containers in the isolator in the sterile state;
a step of conveying the culture containers housing the cells into an incubator and of starting the cell-culture; and
a step of conducting required tests on the cells in the test containers.

2. The method for preparing a therapeutic agent according to claim 1, **characterized in that**
the collection container housing the cells is conveyed into a pass box connected to the isolator, has an outer surface decontaminated in the pass box and then, is conveyed into the isolator; and
the test container is taken out of the isolator through the pass box and then, the required tests are conducted on the cells in the test container.

3. The method for preparing a therapeutic agent according to claim 2, **characterized in that**
the cells are a bone marrow fluid sampled from a patient with decompensated cirrhosis;
the method comprises a step of adding an erythrocyte sedimentation agent to the cells pipetted into the culture container from the collection container so that erythrocytes are precipitated and then collecting supernatant, a step of concentrating a bone marrow cell-containing fraction for liver regeneration from the collected supernatant, and a step of pipetting the concentrated bone marrow cell-containing fraction for liver regeneration into another culture container; and
the another culture container is conveyed into the incubator and the cell-culture is started.
